Europäisches Patentamt

European Patent Office

Office européen des brevets

(19)

(11) Publication number : **0 508 672 A2**

## (12) EUROPEAN PATENT APPLICATION

(21) Application number : 92302867.4

(22) Date of filing : 01.04.92

(51) Int. Cl.⁵ : **C12N 15/16,** C12P 21/02, A23K 1/16, A23K 1/18, C07H 21/04, C12N 1/21, // (C12N1/21, C12R1:19)

(30) Priority : 01.04.91 JP 142251/91

(43) Date of publication of application : 14.10.92 Bulletin 92/42

(84) Designated Contracting States : DE FR GB SE

(71) Applicant : NIPPON OIL CO. LTD.
3-12, Nishi Shinbashi 1-chome
Minato-ku Tokyo (JP)

(72) Inventor : Sakata, Shusaku
2-228-1152, Kosugi-cho, Nakahara-ku
Kawasaki-shi, Kanagawa-ken (JP)

Inventor : Hirano, Tetsuya
1-3-1-202, Ikejiri, Setagaya-ku
Tokyo (JP)
Inventor : Urano, Akihisa
3-34-3, Matsubara, Setagaya-ku
Tokyo (JP)
Inventor : Kawauchi, Hiroshi
124-103, Sugishita, Okkirai, Sanriku-cho
Kesen-gun, Iwate-ken (JP)
Inventor : Mizuta, Haruyoshi
Nisseki AP No. 1-4, 1958-1, Higashi-Toyoi
Kudamatsu-shi, Yamaguchi (JP)

(74) Representative : Ritter, Stephen David et al
Mathys & Squire 10 Fleet Street
London EC4Y 1AY (GB)

(54) **Fish prolactin.**

(57) Disclosed herein are methods and means of producing novel prolactins derived from the pituitary gland of fishes belonging to the order Pleuronectina, the family Paralichthydae, which are useful in the growth promotion for fishes, by genetic engineering using cloned genes.

EP 0 508 672 A2

FIELD OF THE INVENTION

This invention relates to a novel prolactin of fishes, and, more particularly, to a prolactin derived from pituitary gland of fishes belonging to the order Pleuronectina, the family Paralichthydae, a gene coding its amino acid sequence, a recombinant vector integrating said gene, a transformant transformed by said recombinant vector, a new prolactin protein produced by utilizing said transformant and a process for producing the same.

This invention also relates to a process of growth promotion for animals including fishes using the said new prolactin, and further relates to a feed for aminals including fishes, containing a prolactin of fishes belonging to the order Pleuronectina, the family Paralichthydae.

BACKGROUND OF THE INVENTION

A number of growth hormones from fish and mammals have been currently isolated and many of them have been elucidated in regard to even the structure of genes thereof. As these growth hormones could have a growth promotion effect, they have the potential to be applied to growth promotion of fishes and domestic animals, increase in their edible portions in the field of fish farming and livestock raising. And further a growth hormone has been developed in human beings to exert such effects as usefulness in the treatment of dwarfism or nanism.

On the other hand, as a protein similar in its amino acid sequence to the growth hormone there is known the existence of prolactin which is secreted from the lobus anterior hypophyseos of the pituitary gland, like the growth hormone. Heretofore, prolactin has been isolated from many kinds of animals, including quadruped animals and fishes. Generally, prolactin is considered to be a hormone having a wide action related to reproduction such as, for example, secreting milk in mammals at large, exhibiting a luteotropic action, and forming pigeon's milk in birds. In lower vertebrates, prolactin is known to exhibit a metamorphosis controlling action and a growth action in the amphibia. In fishes, moreover, prolactin is considered to participate in the adjustment of osmotic pressure at the time of adaptation of euryhaline fishes and fresh-water fishes to fresh water [Clarke and Bern, 1981, Comparative endocrinology of prolactin "Hormonal Proteins and Peptides" (C. H. Li ed.), Vol.8, pp.105-197, Academic Press, New York]. In marine fishes, although the existence of prolactin is known from immunotissue chemical studies, no study has been made about its isolation and physiological action.

According to a report of Specker et al. [Specker et al., 1985, in Prolactin-Brain and clinical correlates, eds. MacLeod et al., (Liviana, Padoa, Italy), Fidia Research Series, Vol.1, pp.427-436), prolactin of tilapia which is a euryhaline fish has a growth promoting activity higher than that of growth hormones. As mentioned above, moreover, prolactin is known to participate in the control of metamorphosis and growth in the amphibia. Also in fishes, for example fishes belonging to the order Pleuronectina examples are known wherein dramatic metamorphosis is performed. However, scarcely any studies have been made about the role of prolactin for metamorphosis and growth in the fry period of that fish.

Then, the present inventors have made earnest studies upon their considerations that there may be found any growth factor having a new physiological activity, paticularly a growth promoting activity, in marine fish, and that there may be not only a great scientific development but also a higher industrial usefulness, for example, application to growth promoting agents in aquaculture, if such a substance could be found and obtained in a large amount. As a result of these studies, they have derived a novel prolactin from the pituitary gland of flounder or halibut and further developed a process for producing the same in a large amount, with a successful cloning of the gene coding the amino acid sequence of such a prolactin.

The present inventors have also found that the prolactin from flounder or halibut is effective in promoting growth of animals, including fishes.

SUMMARY OF THE INVENTION

It has been found that there is a novel prolactin in the pituitary gland of fishes belonging to the order Pleuronectina, the family Paralichthydae, in particular, flounders, which can be isolated and purified, and there can be obtained a gene which codes the amino acid sequence and the gene can be successfully expressed by cloning the gene by means of a gene recombination technique and the desired prolactin in a high purity can be easily produced in a large amount. This invention has been further completed by confirming that both the prolactin obtained from the pituitary gland as described above and the prolactin obtained according to a gene recombination technique as described above can exert a satisfactory growth promoting activity on animals, in particular, fishes.

According to this invention, there is provided a novel prolactin derived from pituitary gland or hypophysis of fishes, and, more particularly, a prolactin derived from pituitary gland of fishes belonging to the order Pleuronectina, the family Paralichthydae, a gene coding its amino acid sequence, a recombinant vector integrating said gene, a transformant transformed by said recombinant vector, a new prolactin produced by utilizing said transformant and a process for producing the same.

This invention also relates to a process of growth promotion for animals including fishes using the said new prolactin protein.

## BRIEF DESCRIPTION OF THE DRAWINGS

FIG. 1 shows the elution pattern for the pituitary gland of flounder by a Sephadex G-75 column chromatography.

FIG. 2 shows the elution pattern for prolactin by a high performance liquid chromatography.

FIG. 3 shows restriction maps and sequence strategies for the gene coding the amino acid sequence of prolactin, wherein the figure in parentheses( ) shows the base number from 5′ terminal.

FIG. 4 shows the DNA base sequence and amino acid sequnece for the coding of the present prolactin.

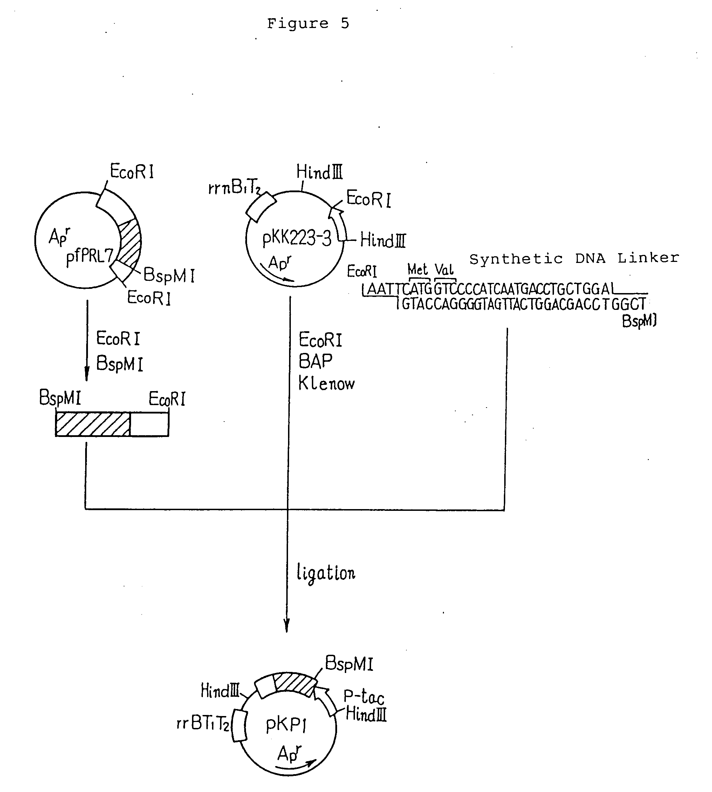

FIG. 5 shows the procedures for constructing recombinant plasmid pKP1.

## DETAILED DESCRIPTION OF THE INVENTION

The present invention provides prolactins derived from the pituitary gland of fishes belonging to the order Pleuronectina, the family Paralichthydae, more specifically Paralichthys, genes which code for the amino acid sequence of the above prolactin, recombinant vectors integrated with said gene and transformants containing said recombinant vector integrated with said gene.

The present invention further provides processes for producing prolactins derived from the pituitary gland of fishes belonging to the order Pleuronectina, the family Paralichthydae which comprises incubating and multiplying the transformant transformed by the recombinant vector, said vector being integrated with the gene coding for the amino acid sequence of the said prolactin, and recovering the said prolactin as produced, and processes of growth promotion for fishes which comprises feeding fishes with the said prolactin to promote growth of fishes.

The present invention provides specifically prolactins which are proteins having the following physicochemical properties;

(1) Molecular weight: SDS-PAGE about 22,000 daltons,

(2) Amino acid composition

| Amino acid | Residue number | Amino acid | Residue number |
|---|---|---|---|
| C y s | 4.0 | P r o | 12.5 |
| A s p | 12.4 | T y r | 0.9 |
| G l u | 19.8 | V a l | 7.2 |
| S e r | 24.0 | M e t | 2.7 |
| H i s | 6.3 | I l e | 8.5 |
| G l y | 9.6 | L e u | 27.1 |
| A r g | 11.5 | P h e | 8.3 |
| T h r | 6.8 | T r p | ND |
| A l a | 16.6 | L y s | 7.5 |

(3) 22 Amino acid residues at N-terminal end having the following sequence:

Val-Pro-Ile-Asn-Asp-Leu-Leu-Asp-Arg-Ala-

Ser-Gln-Arg-Ser-Asp-Gln-Leu-His-Ser-Leu-

Ser-Thr

The present invention further provides prolactin proteins which are polypeptides obtained by a gene recombination procedure, contain the following amino acid sequence in the molecule and have a prolactin activity:

```
Val Pro Ile Asn Asp Leu Leu Asp Arg Ala Ser Gln Arg Ser Asp
 1                                     10

Gln Leu His Ser Leu Ser Thr Thr Leu Ser Gln Glu Leu Asp Ser
             20                                          30

His Phe Pro Pro Ile Gly Arg Val Ile Met Pro Arg Pro Ser Met
                                 40

Cys His Thr Ser Ala Leu Gln Thr Pro Asn Asp Lys Thr Gln Ala
             50                                          60

Leu Gln Val Ser Glu Ser Glu Leu Leu Ser Leu Ala Arg Ser Leu
                             70

Leu Gln Ala Trp Ala Asp Pro Leu Ser Ala Leu Ser Ser Ser Ala
             80                                          90

Phe Ser Leu Pro His Pro Ala Gln Ser Ser Ile Phe Asn Lys Val
                                 100

Arg Glu Met Gln Glu His Ser Lys Asn Leu Gly Asp Gly Leu Asp
             110                                         120

Ile Leu Ser Gly Lys Met Gly Glu Ala Gly Gln Ala Leu Ser Ser
                             130

Leu Pro Phe Arg Gly Asn Asp Val Gly Gln Asp Arg Ile Ser Lys
             140                                         150

Leu Ile Asn Phe His Phe Leu Leu Ser Cys Phe Arg Arg Asp Ser
                             160

His Lys Ile Asp Ser Phe Leu Lys Val Leu Arg Cys Arg Ala Ala
             170                                         180

Asn Thr Gln Pro Glu Met Cys
                 187
```

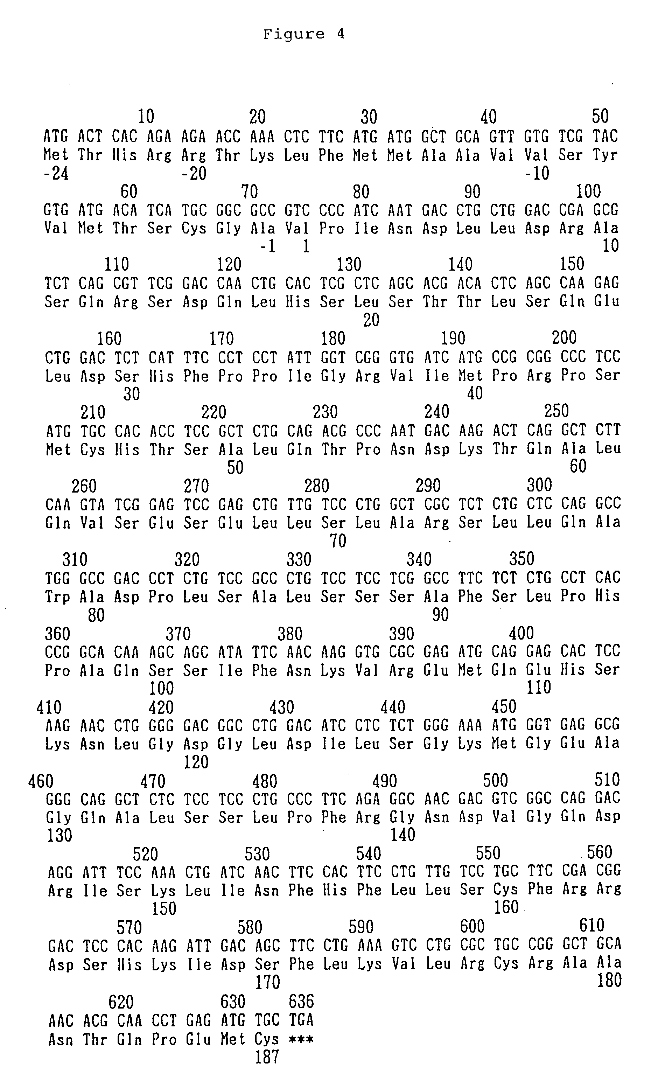

A representative polypeptide has the amino acid sequence as shown in Fig. 4.

A representative gene which codes for the amino acid sequence of the prolactin derived from the pituitary gland of fishes belonging to the order <u>Pleuronectina</u>, the family <u>Paralichthydae</u> is DNA having the base sequence as shown in Fig. 4.

Another representative gene which codes for the amino acid sequence of the prolactin derived from the pituitary gland of fishes belonging to the order <u>Pleuronectina</u>, the family <u>Paralichthydae</u> is a synthetic DNA corresponding to the amino acid sequence from the 1th to 13nd from N-terminal end, of the amino acid sequence of the prolactin derived from the pituitary gland of fishes belonging to the order <u>Pleuronectina</u>, the family <u>Paralichthydae</u>.

The prolactins according to the present invention can be isolated and purified according to various methods as described below.

The said method may be accomplished, for instance, by applying a method which is known for isolation and purification of a variety of proteins, starting from the tissues or cells containing the above-mentioned prolactin, for example, the pituitary gland of flounders, i.e. fishes belonging to the order <u>Pleuronectina</u>, the family <u>Paralichthydae</u>.

As the methods for the isolation and purification of proteins, there may be mentioned solubilization by a homogenizer, ultrasonic cell breaking and the like; extraction with a buffer containing various salts; solubilization or precipitation with an acid or alkali; extraction or precipitation with an organic solvent, salting-out with ammonum sulfate and the like; dialysis; ultrafiltration using a membrane filter and the like; gel filtration chromatography; ion exchange chromatography; reversed-phase chromatography; countercurrent chromatography; high performance liquid chromatography; isoelectric point; or gel electrophoresis and so on and they may be employed alone or in any optional combination therewith.

A method for the isolation and purification of the present prolactin may be illustrated in detail as below. The pituitary gland was obtained from flounder in a conventional manner, immediately frozen by using liquid nitrogen or dry ice and stored at about -80 °C up to its use. Then, the pituitary gland is minced in a hydrochloric acid-acetone solution (1:28) under cooling, and then centrifuged.

The precipitate thus obtained is extracted in 80° acetone and the extract is added dropwise into acetone which has been cooled to -20°C , then the precipitate is obtained after centrifugal separation. After freeze-drying, the precipitate is dissolved in a suitable buffer solution and then subjected to gel filtration chromatography, e.g. chromatography with a Sephadex G-100 column. Eluted fractions can be measured and collected by monitoring the absorbance at 280 nm. Then, of the fractions thus obtained, the desired fractions containing prolactin are freeze-dried.

Thereafter, this purified product is dissolved in a suitable buffer solution and then subjected to a high performance liquid chromatography, for example, a high performance liquid chromatography using a chemically modified silica gel such as ODS 120T as a carrier. The fractions containing the desired prolactin are collected by monitoring the eluates with the absorbance at 220 nm. The prolactin thus obtained can be determined for its properties by various physicochemical analyses.

As a result of electrophoresis with SDS polyacrylamide gel, the present prolactin is presumed to have a molecular weight of about 22,000 daltons. The protein obtained by the foregoing high performance liquid chromatography produces a single band in electrophoresis with SDS polyacrylamide gel.

The prolactin thus isolated and purified is hydrolyzed with an acid, e.g. hydrochloric acid, or a proteolytic enzyme, e.g. pepsin, chymotrypsin, or carboxypeptidase, and the peptide fragments thus obtained are subjected to a chromatography, e.g. ion exchange chromatography, and can each be analyzed for its amino acid composition and determined for its amino acid sequence.

Analysis for the amino acid composition of the present prolactin will now be explained in more detail. First, the purified prolactin is hydrolyzed with hydrochloric acid and then reacted with phenyl isothiocyanate (PITC) to convert the amino acids into the corresponding phenylthiocarbamyl derivatives, then the derivatives are quantitatively determined by a reverse-phase high performance liquid chromatograph (PITC method).

The amino acid composition of the present prolactin thus obtained is as shown in Table 1.

As seen from this table, the present prolactin has prolactin-like properties in that it has four cysteines and is rich in serine and leucine.

For determining the amino acid sequence of the present prolactin, there may be used a conventional peptide sequencer, with a gas-phase peptide sequencer being particularly preferred.

Using such a peptide sequencer, it is possible to determine the amino acid sequence from N-terminal of the protein. The sequence of 22 amino acid residues at N-terminal end of the present prolactin as determined by such procedures is as by such procedures is as shown below.

```
Val-Pro-Ile-Asn-Asp-Leu-Leu-Asp-Arg-Ala-

Ser-Gln-Arg-Ser-Asp-Gln-Leu-His-Ser-Leu-

Ser-Thr
```

The gene, which may code for the amino acid sequence of the present prolactin, may be prepared according to a variety of methods as disclosed below.

As the said methods, there may be mentioned, for instance, a method wherein poly(A)RNA is prepared from the pituitary gland of flounder, fishes belonging to the order Pleuronectina, the family Paralichthydae, which is producing cell for the above-mentioned prolactin, cDNA is synthesized by using the poly(A)RNA as a template and multiplied in host cells with a suitable vector, a clone is selected which contains cDNA coding for the aimed amino acid sequence of the prolactin and the gene is isolated from the vector of said clone; a method wherein a suitable DNA probe is synthesized upon the finding obtained as a result of the amino acid sequence analysis of the prolactin obtained from fishes according to the present isolation and purification method, a gene library is investigated using such a probe to select the clone containing cDNA coding for the amino acid sequence of the desired prolactin and the corresponding gene is isolated from said clone; a method wherein a nucleic acid is chemically synthesized upon DNA of the gene coding for the amino acid sequence of the present prolactin according to a conventional method, for example, a phosphotriester method (Tetrahedron, 34, 3143 (1978), Adv. Carbohydr. Chem. Boiochem., 36, 135 (1979), Nucleic Acids Res., 10, 2597, 6553 (1982)), a phosphoramidite method (Nature, 310, 105 (1984)) and others; or any combination of these methods.

A method for preparing the said gene from the above flounder pituitary gland will be illustrated in detail below.

In order to obtain the gene of this invention, it is necessary to extract RNA from flounder pituitary gland, and one may preferably employ any of raised and natural flounder. The pituitary gland may be isolated from flounders in a conventional manner and the pituitary gland thus isolated is immediately frozen by the use of liquid nitrogen, dry ice- acetone and the like and, as required, may be stored at about -80°C until it is to be used.

From the pituitary gland obtained as above, RNA may be extracted in a conventional manner, which may include separation of polysome, utilization of sucrose density gradient centrifugation or electrophoresis. As the extraction method for RNA from the said pituitary gland, there may be mentioned a guanidine· thiocyanate - cesium chloride method wherein CsCl density gradient centrifugation is effected after treatment with guanidine· thiocyanate (Chirgwin, et al., Biochemistry, 18, 5294 (1979); a method which comprises treating with a surface active agent in the presence of an inhibitor of ribonuclease using a vanadium complex and then treating with phenol (Berger, et al., Biochemistry, 18, 5143 (1979); a guanidine · thiocyanate - hot phenol method; a guanidine· thiocyanate - guanidine hydrochloric acid method; a guanidine· thiocyanate - phenol· chloroform method; a method which comprises treating with guanidine· thiocyanate and then treating with lithium chloride to precipitate RNA; and others.

The RNA extraction method from the said pituitary gland will be illustrated in detail as below.

The frozen pituitary gland may be first solubilized by mechanically mincing in a guanidine- thiocyanate solution, lithium chloride is then added and, where necessary, centrifugation is done to obtain cellular RNA as precipitate. Thereafter, the precipitate thus obtained may be, where necessary, extracted with phenol and then precipitated with ethanol to give RNA as a precipitate.

From the RNA thus obtained, one may further purify the RNA having a polyA chain in a conventional manner. As such method, there may be advantageously employed an affinity column chromatography using oligo-dT cellulose and others.

The poly(A)RNA thus obtained may be employed as a template for the synthesis of a double-stranded DNA (cDNA) using a reverse transcriptase. The cDNA may be synthesized according to any conventional methods. First, the first stranded DNA is synthesized with oligo(dT) or vector primer as a primer using, for example, any reverse transcriptase derived from, e.g. AMV, MMLV, RSV and the like and then treated with Klenow fragment to form the double-stranded DNA. Then, it is treated with T4 DNA ligase and then with S1 nuclease or treated with S1 nuclease and then with terminal transferase. Alternatively, the first stranded DNA-containing product obtained using the said reverse transcriptase is subjected to action by DNA polymerase I, robonuclease H and E. coli DNA ligase. As such methods, there may be mentioned those as disclosed in Methods in Enzymology, Vol. 152, 307 ~ 335, Ed. by Shelby L. Barger et al., Academic Press, Inc. (1987), but it is practically convenient for this cDNA synthesis to utilize any cDNA synthesis kits commercially available form, e.g. Amersham Japan Ltd.

The cDNA thus obtained may be inserted into a suitable vector which is then amplified by introducing into a suitable host and cloning, a recombinant host containing cDNA coding for the amino acid sequence of the desired prolactin is selected by conducting a screening and then the DNA coding for the amino acid sequence of the said prolactin can be isolated from the plaque or colony of such recombinant host.

As the vector which may be employed for insertion of the said cDNA, there may be mentioned a variety of plasmid vectors commonly employed, and one may prefereably employ, for example, pBR322, λ bacteriophage vectors, λ gt 10, λ gt 11 and the like.

And there is no particular limitation to the host which may be employed herein, provided that the said recombinant vector may accomplish autoreplication, and, for example, E. coli may be preferably employed as the host. Particularly when λ gt 10 DNA is employed as the said vector, E. coli NM514 strain may be preferably employed as a host.

In inserting the said cDNA into a vector, there may be employed any conventional methods in which a restriction site produced by using the identical restriction enzyme is utilized, any synthetic linker or adaptor is added where necessary or a homopolymer is added.

For instance, more specifically, EcoRI linker may be added to cDNA at both terminal ends using T4 ligase, digestion is done with restriction enzyme EcoRI, and the vector fragment digested with the same restriction enzyme EcoRI is ligated, thereby the desired recombinant vector being obtained.

The recombinant vector thus obtained may be introduced into a host by any methods commonly employed.

As the methods, there may be mentioned, for example, a method wherein the vector may be incorporated into competent cells prepared in the co-existence of $CaCl_2$ or RbCl according to Hanahan et al., J. Mol. Biol., 166, 557 (1983); a method wherein the host in a suitable propagative phase is treated with $MgSO_4$ and the like and then a recombinant phage vector is infected; and others.

Thereafter, the recombinant containing DNA coding for the amino acid sequence of the prolactin is selected by screening. In this selection, there may be applied any well-known various methods, for example, a colony or plaque hybridization method using chemically synthesized oligonucleotide probe; a hybridization·translation assay method; a plus-minus method; and others may be advantageously applied.

More specifically, the DNA of the recombinant plaque is fixed on a filter of, e.g. a nylon membrane and then reacted with a labeled probe to select a recombinant having the DNA sequence to be selectively bound to the said probe.

The probe as used herein is indicated to be a nucleic acid sequence having a complementary sequence to the aimed DNA sequence and it may be RNA or DNA and of a chemically synthesized or natural type or of a type obtained according to a recombinant DNA procedure. The said DNA sequence as chemically synthesized by application of well-known methods may be generally and preferably employed.

In synthesizing the probe DNA sequence chemically, the sequence may be determined upon the amino acid sequence of the aimed prolactin, in particular, the amino acid sequence of the flounder prolactin.

The DNA base sequence of the gene according to this invention as obtained from the selected recombinant as above may be determined according to Maxam-Gilbert method, Dideoxy method, e.g. dideoxynucleotide chain termination method (Sanger, Science, 214, 1205 (1981); Methods in Enzymology, 65, 560-580 (1980); Messing. J. et al., Nucleic Acids Res., 9, 309 (1981) and others.

The DNA sequence and amino acid sequence, which contain the gene of the present prolactin, particularly, the prolactin derived from flounder, as determined above are as shown in Fig. 4. The sequence includes the DNA sequence and amino acids of the precursor for the prolactin from flounder. In this DNA sequence, the first to 72nd amino acid sequence sites correspond to signal peptides, while the 73rd to 633rd amino acid sequence sites correspond to mature proteins. It has been elucidated that said prolactin from flounders is first bio-synthesized as the said precursor and then the signal peptide sites are removed to form secretory proteins.

The amino acid sequence determined upon this DNA sequence is in perfect agreement with the amino acid sequence of the said prolactin already determined upon the natural product and hence the said cDNA obtained from the recombinant has been confirmed to code for the amino acid sequence of the prolactin from flounder.

Also, the DNA base sequence has been confirmed to have a significant homology with those growth hormones discovered in the past.

According to gene recombination techinques, there can be carried out deletion, insertion, addition and ligation of DNA chains including even substitution of bases in DNA chains in a conventional manner and, therefore, the present gene relates not only to the gene having the base sequence as shown in Fig. 4, but also to alterations and modifications thereof as mentioned above within the scope not departing from the present objects.

Typical of such methods are the methods known as oligonucleotide directed mutagenesis methods such as, for example, the methods described in M. Smith and S. Gillam, Genetic Engineering (J. K. Seltow and A. Hollaender eds.), Vol.3, p.1 (1981), and Methods in Enzymology, (1987), Academic Press, CA, as well as those described in the literatures cited therein.

The most preferred alteration and modification of the gene having the base sequence shown in Fig. 4, which fall within the present scope, may be an increase in stability and biological activity of the aimed protein.

The present gene coding for the amino acid sequence of the prolactin may be produced in a large amount from the above-mentioned recombinant vector using a restriction enzyme according to a conventional method and the gene thus obtained may produce a protein having the amino acid sequence of the prolactin in a high purity and a large amount according to gene recombination techniques.

In utilizing the present gene as above, there may be employed various procedures commonly employed for usual gene recombination techniques.

The present gene may be recombined to a suitable expression vector, tranformation is carried out by introducing the recombinant vector into a suitable expression host, the resulting recombinant is incubated to induce a suitable expression, thereby producing the desired protein.

In producing the desired protein in a host using the present gene, the protein may be produced as a mature protein, namely in the form of the signal peptide removed, or alternatively the protein be produced by secretion from host cells by utilizing the said signal peptide as such or by adding any signal peptide adaptive to a suitable host cell or the like.

In this instance, initiation codon and stop codon would be required for introducing into the gene DNA sequence to be employed therefor and, where necessary, they may be added by employing any of well-known methods.

As the signal peptides adaptive to the said host cells and the like which may be preferably employed, there may be mentioned those from cellular secretory protein precursors and, for instance, those relating to gram-negative and gram-positive bacteria such as E. coli β -lactamase, phosphated protein, alkaline phosphatase, neutral protease from the genus Bacillus and the like.

And further, expression of the said gene may be accomplished in a host cell together with, e.g. interferon, interleukin 2, proinsulin, various hormones.

In recombination of the present gene into an expression vector, there may be employed any usual methods employed for well-known gene recombination techniques, e.g. ligation with a variety of restriction enzymes.

As the expression vectors which may be utilized herein, there may be employed without specific limitation any of those capable of doing autoreplication in a host, and there may be preferably utilized those vectors wherein a replicating origin, a selective marker, a promotor, a RNA splicing site, a polyadenylated signal and the like may be involved.

As the vectors, there may be mentioned those derived from various bacteria, bacteriophage and animal virus, and there may be mentioned various viral vectors, various plasmid vectors, cosmid vector, shuttle vector and others.

As the vectors, there may be also mentioned E. coli, in particular, EK type plasmid vector, λ gt type phage vector, Pseudomonas aeruginosa derived vector, Bacillus subtilis derived vector, yeast derived vector, SV40 derived vector and the like.

As the promotor which may be employed for the above vectors, there may be mentioned those poromotors well known to those skilled in the art, such as tryptophan (trp) promotor, lactose (lac) promotor, tryptophan·lactose (tac) promotor, bacteriophage derived lambda ( λ ) $P_L$ promotor and others.

These gene controlling sequences may be optionally combined or chemically modified to be intergrated into a suitable vector, whereby the vector for expression of the present gene may be constructed.

Into the present gene expression vector may be further integrated plural genes of this invention to carry out expression.

The gene expression vector, which codes for the amino acid sequence of the prolactin may be introduced into a suitable host, in particular, a host cell according to any well-known method to transform the said host cell and then the host cell thus transformed may be multiplied by incubation to obtain a large amount of the cell having a productivity of the peptide having the amino acid sequence for the prolactin, which is referred to as "transformant".

As the host, particularly, the host cell which may be employed herein, there may be any of E. coli; other gram-negative bacteria than E. coli; gram-positive bacteria, e.g. Bacillus subtilis or actinomycetes; yeast; eucaryotic cell, e.g. animal and plant cells, and E. coli may be preferably employed.

For introduction of the present gene expression vector into the above-mentioned host and transformation there with, there may be employed any methods commonly employed in the field of gene recombination techniques, for example, admixture of competent cells with the said vector, conversion of the cell to protoplast and subsequent incorporation of the said vector bound to a carrier or coprecipitation with calcium phosphate and others.

The transformant thus obtained, after multiplication under the condition to inhibit the expression of its exogenote or vector, may induce the expression of the said exogenote or vector.

Multiplication or incubation of the transformant may be carried out by employing conventional, various media for cell culture and, as examples thereof, there may be mentioned those media which contain, e.g. carbon sources, nitrogen sources, vitamins, nucleic acid bases, inorganic salts and the like and are optionally supplemented with meat extract, peptone, Casamino acid, yeast extract, fish solubles, potato, malt extract, milk, blood, serum, hormones, antibiotics and others. Generally, commercially available media may be preferably employed as such or after suitably modified.

In multiplication or incubation of the said transformant, one may optionally determine the condition such as pH, temperature, aeration, stirring, frequency to exchange a medium and the like, which would be suitable for the growth of the said transformant.

The protein upon the expression of the present gene, which is produced by multiplication or incubation of the said transformant or by induction of the expression of the said gene, may be isolated and recovered according to usaual procedures.

As the isolation and recovery method, there may be mentioned, for example, ultrasonic disrupting of cells, mechanical mincing, freeze-drying and melting, osmotic pressure shock disrupting and others, as well as precipitation from cultured supernatant, e.g. using a protein-precipitating agent for separation.

In addition to the above-mentioned separation methods, the aimed protein may be further purified by application of various procedures for isolation and purification generally and widely employed, utilizing physiochemical or chemical properties of the protein.

As the isolation and purification methods, there may be employed precipitation with such protein-precipitating agents as ammonium sulfate as described above, as well as ultrafiltration, gel filtration, adsorption chromatography, ion exchange chromatography, affinity chromatography, high performance liquid chromatography, electrophoresis or any combination therewith.

Further, the protein obtained from the transformant can be activated by a suitable method known in this field, including denaturation and regeneration, e. g. treatment with a guanidine solution and subsequent dialysis.

The present prolactin, in particular, the prolactin derived form the pituitary gland of fishes belonging to the order Pleuronectina, the family Paralichthydae can be easily confirmed for its biological activity according to the following method: Juvenile flounder are individually selected and the protein is given intraperitoneally or intramuscularly every 4 days at a dose of 0.01 to 0.1μ g per gram of fish body weight and then body weight gain and body length gain of fishes can be measured to confirm its biological activity.

The foregoing is merely illustrative of the invention and is not intended to limit the invention to the disclosed compounds. Variations and changes which are obvious to one skilled in the art are intended to be within the scope and nature of the invention.

The transformant Escherichia coli fPRL7, FERM BP-3333 (January 5, 1990) according to the procedures described in Example 8 was deposited at the Fermentation Research Institute, Agency of Industrial Science and Technology, Ministry of International Trade and Industry, Japan (FRI) under the Budapest Treaty. Its accession numbers on the deposit dates are shown above (The deposit dates are indicated in parenthesis).

In the specification and drawings of the present application, the abbreviations used for bases, amino acids and so forth are those recommended by the IUPAC-IUB Commission on Biochemical Nomenclature or those conventionally used in the art. Amino acids for which optical isomerism is possible are, unless otherwise specified, in the L form.

## Example

The invention is further illustrated but in no way limited by the following examples.

## Example 1

## Exatraction of Flounder Prolactin

From 170 flounder aged one year were excised 1.2 g of the pituitary gland, which was immediately frozen in liquid nitrogen and then stored at a temperature of -80°C until its use. The pituitary gland (1.2 g) was minced in 3 ml of a hydrochloric acid-acetone (1:28) solution under ice-cooling by means of a glass homogenizer. After subsequent acid-acetone extraction on ice for 1 hour, the pituitary gland was centrifuged at 0°C, 10,000 rpm, for 10 minutes by means of a centrifuge rotor, himac SCR20B (Hitachi Ltd., Japan). The resulting precipitate was homogenized in 3 ml of 80 % acetone, then extracted on ice for 1 hour and centrifuged, together with the supernatant liquid obtained in the above centrifugation, at 0°C, 10,000 rpm, for 10 minutes by means of the aforesaid centrifuge. The extract thus obtained was dropwise added into acetone held at -20°C and thereby precipitated. After removal of the supernatant liquid, the precipitate was freeze-dried. The product thus obtained weighed about 26 mg.

## Example 2

## Purification of Flounder Prolactin

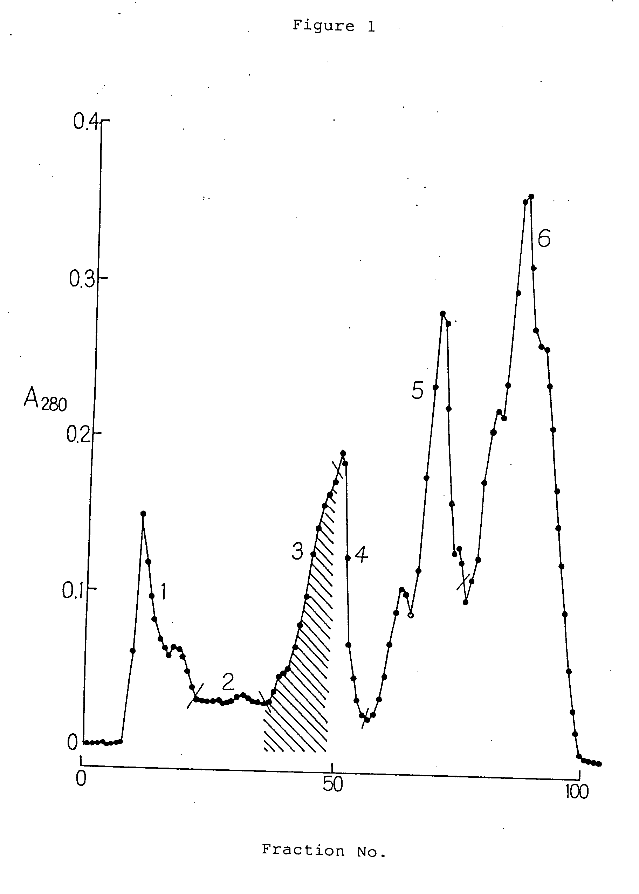

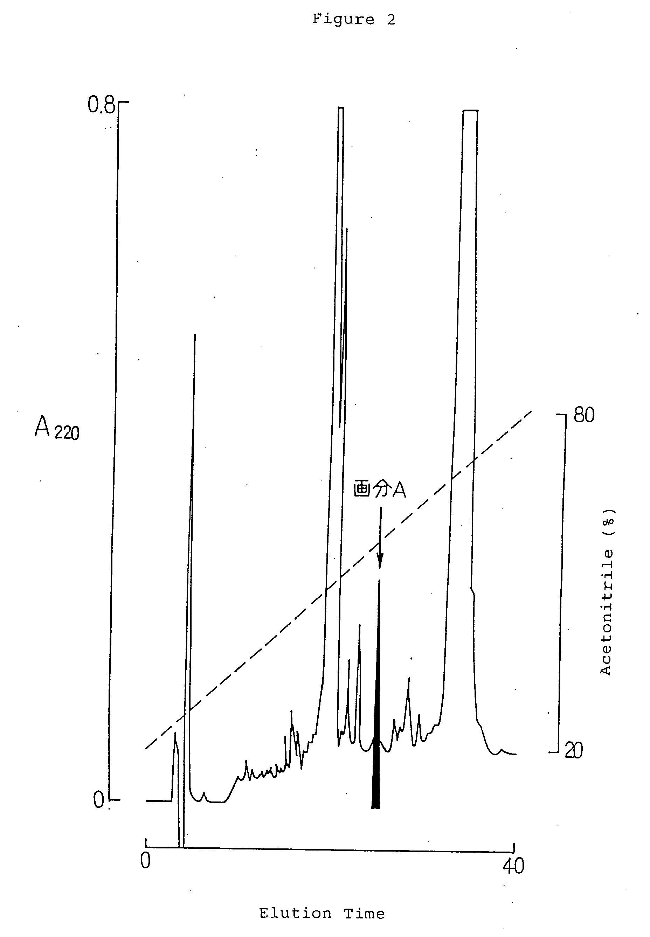

The power obtained above was dissolved in 3 m$\ell$ of 0.1N acetic acid and the solution was fractionated through a column of Sephadex G-75 (Pharmacia Ltd.) equilibrated with the same solution. Under elution conditions of a prestream of 70 ml and a flow rate of 15 ml/hr, 3 ml of the eluate was dispensed for each test tube, and the protein content was measured by determining the absorbance at 280 nm to afford 6 fractions (see Fig. 1). The fraction No. 3 shown in Fig. 1 was freeze-dried to give 5.8 mg of a white powder, which was then purified by a high performance liquid chromatography (HPLC) using a column of ODS120T. The elution conditions for HPLC were set to be 0.1% trifluoroacetic acid, acetonitrile 20-80% linear gradients, a column temperature of 40°C and a flow rate of 1 ml/min., and the protein content was measured by determining the absorbance at 220 nm to obtain flounder prolactin (Fig. 2). The fraction A thus obtained exhibited the following physicochemical properties.

Example 3

Determination of Molecular Weight

The fraction A obtained above was developed by electrophoresis with SDS polyacrylamide gel to establish that the flounder prodactin has a molecular weight of 22,000 At the same time, the fraction A showed a single band.

As a standard molecular weight market there was used a colored molecular weight marker LMW kit E (Pharmacia Ltd.), molecular weights 94,000; 67,000; 43,000; 30,000; 20,000; 14,000.

Example 4

Analysis of Amino Acid Composition

The flounder prolactin ($0.1\mu$ g) was hydrolyzed with 20 $\mu$ l of 6N hydrochloric acid containing 0.6% phenol at 110°C for 18 hours and then amino acids were reacted with phenyl isothiocyanate (PITC) to converted to the corresponding phenylthiocarbamyl derivatives. Thereafter, the derivatives were quantitatively determined by a reversed-phase HPLC (PITC method). The HPLC parameter was a column TSK ODS 80T (Tosoh Corporation, Japan, 0.4 x 25 cm). The elution parameters were as follows: Solution A, 0.14M sodium acetate pH 5.4: acetonitrile = 90 : 10 (containing 0.05% triethylamine), Solution B, 60% acetonitrile; initial Solution A 100% - Solution B 80%; 20 minutes; linear gradient; column temperature of 45°C, flow rate of 1 ml/min. The amino acid composition was determined by measuring the absorbance at 254 nm. The results obtained are as shown in table below.

The results are shown in the following Table.

## Table 1

### Amino acid composition

| Amino acid | Residue number | Amino acid | Residue number |
|---|---|---|---|
| C y s | 4.0 | P r o | 12.5 |
| A s p | 12.4 | T y r | 0.9 |
| G l u | 19.8 | V a l | 7.2 |
| S e r | 24.0 | M e t | 2.7 |
| H i s | 6.3 | I l e | 8.5 |
| G l y | 9.6 | L e u | 27.1 |
| A r g | 11.5 | P h e | 8.3 |
| T h r | 6.8 | T r p | ND |
| A l a | 16.6 | L y s | 7.5 |

The flounder prolactin has 4 cysteines and is rich in leucine and serine, thus exhibited prolactin-like properties.

## Example 5

### Determination of N-Terminal Amino Acid Sequence

The said flounder prolactin (10 μ g) was determined for its N-terminal amino acid sequence by means of a gas-phase peptide sequencer (Shimazu Corporation, Japan).

The sequence of the 22 amino acids at the N-terminal side as determined is as shown below:

```
Val-Pro-Ile-Asn-Asp-Leu-Leu-Asp-Arg-Ala-

Ser-Gln-Arg-Ser-Asp-Gln-Leu-His-Ser-Leu-

Ser-Thr
```

## Example 6

### Preparation of cDNA Library of Flounder Pituitary Gland

The whole RNA was extracted from flounder pituitary gland by employing a RNA extraction kit (Amersham Ltd.).

The flounder pituitary gland (1.0 g) was minced in 20 mℓ of a guanidine. thiocyanate solution (a solution comprising a guanidine. thiocyanate and 8 % β -mercaptoethanol) on an ice bath, 0.3 volume of ethanol was added and the mixture was then centrifuged and precipitated at 0°C at 10,000 rpm for 5 minutes by means of a centrifuge rotor (Hitachi Ltd., Japan, himac SCR20B). The supernatant was romoved, the precipitate was suspended in the above-mentioned guanidine. thiocyanate, 30 mℓ of a lithium chloride solution was added and the mixture was allowed to stand at 0°C for 16 hours to precipitate RNA selectively, followed by centrifugation at 10,000 rpm for 90 minutes by means of the said centrifuge rotor. The resulting precipitate was dissolved in 35 mℓ of a lithium chloride solution containing urea, and centrifuged at 4°C and 10,000 rpm for 60 minutes by means of the said centrifuge. After removal of the supernatant liquid, the residue was dissolved in 5 mℓ of RNA buffer and the whole RNA was then purified, in turn, by phenol extraction (extracted twice with ml of phenol, each at room temperature for 60 minutes), chloroform extraction (5 mℓ of chloroform: isoamyl alcohol (24 : 1), mixed for 5 seconds) and ethanol precipitation (precipitated with 20 mℓ of ethanol and 0.5 mℓ of 2 M sodium acetate, pH 5.0, at -20°C for 16 hours and then centrifuged at 4°C and 9,000 rpm for 30 minutes by means of the said centrifuge rotor). The resulting whole RNA was 3.6 mg.

Then, the RNA was dissolved in 1 mℓ of a buffer (0.5 M NaCl, 20 mM Tris, 1 mM EDTA, 0.1 % SDS, pH 7.6) and the SDS solution was passed through an oligo-dT-cellulose column (5′-3′ Prime Ltd., a column volume of 1mℓ ) to purify poly(A)RNA. The poly(A)RNA was obtained in a total amount of 20 μ g from 1 mg of the whole RNA.

Using the poly(A)RNA thus obtained, there was carried out the synthesis of complementary DNA. The synthesis of cDNA was carried out by employing a cDNA synthesis system plus (Amersham Ltd.).

The flounder pituitary gland poly(A)RNA (5 μ g) was dissolved in 25 μ ℓ of a buffer for the synthesis of 1st strand. To the solution were added, in turn, a mixture of deoxynucleoside triphosphates (containing all of dATP, dGTP, dTTP and dCTT, 5 μ ℓ ), 2.5 μ ℓ of 0.17 OD units oligo(dT$_{12-18}$)primer and 100 units of a reverse transcriptase to make up a whole amount to 50 μ ℓ . Reaction was effected at 42°C for 60 minutes to synthesize the DNA complementary to RNA.

Then, to 50 μ ℓ of the resulting reaction mixture were added 93.5 μ ℓ of a buffer for the synthesis of 2nd strand, 115 units of DNA polymerase in E. coli and 4 units of DNA ribonuclease in E. coli to make up a whole amount to 250 μ ℓ . Reaction was effected at 12°C for 60 minutes and then at 22°C for 60 minutes, and heating was applied at 70°C for 10 minutes to stop the reaction. Ten unites of T4 DNA polymerase were added and the reaction was effected at 37°C for 10 minutes to synthesize cDNA with blunt ends.

To the reaction mixture thus obtained were added 20 μ ℓ of 0.25 M EDTA (pH 8.0) to stop the reaction. The reaction mixture was subjected to phenol-chloroform extraction (125 μ ℓ of phenol, 125 μ ℓ of chloroform, extracted twice each several minutes at room temperature) and subsequent ethanol precipitation [precipitated with 500μ ℓ of ethanol over dry ice and then centrifuged at 12,000 rpm for 20 minutes by means of MCX-150 (Tomy Ltd.)] to produce 3 μ g of cDNA.

Then, the resulting DNA complementary to the flounder pituitary gland poly(A)RNA was integrated into λ gt 10 to construct flounder pituitary gland cDNA library. In the construction of the library, one employed the cDNA cloning system λ gt 10 (Amersham Ltd.).

To 10 μ $\ell$ of a buffer for the methylation reaction at EcoRI site involving 1 μ g of flounder pituitary gland cDNA were added 2 μ $\ell$ of a liquid of 20 units of EcoRI methylase and adenosyl methionine to make up a whole amount to 20 μ $\ell$ . Reaction was effected at 37°C for 60 minutes and the reaction was stopped by heating the reaction mixture at 70°C for 10 minutes.

Then, to 20 μ $\ell$ of the resulting reaction mixture were added 3 μ $\ell$ of a ligation buffer, 5 units of T4 DNA ligase and 2 μ $\ell$ (2 μ g) of EcoRI linker to make up a whole amount to 30μ $\ell$ . Reaction was effected at 15°C for 16∼ 20 hours to add EcoRI linker to cDNA.

Thereafter, to 30 μ $\ell$ of the resulting reaction mixture were added 10 μ $\ell$ of an EcoRI digestion buffer and 100 units of restriction enzyme EcoRI to make up a whole amount to 100 μ $\ell$ . Reaction was effected at 37°C for 5 hours to form the EcoRI cohesive ends derived from EcoRI linker at the cDNA terminals.

The eluate obtained by passing the resulting reaction liquid through a gel filtration column attached to the kit was subjected to ethanol precipitation (precipitated with 800μ $\ell$ of ethanol at -20°C for 2 hours and then centrifuged at 12,000 rpm for 30 minutes by means of MCX-150 (Tomy Ltd.)) to produce cDNA having the EcoRI cohesive ends derived from EcoRI linker.

Then, to 5 μ $\ell$ of a liquid containing 300 ng of the resulting cDNA having the EcoRI cohesive ends derived from EcoRI linker were added 1 μ $\ell$ of a ligation buffer, 2.5 units of T4 DNA ligase and 2 μ $\ell$ (1 μ g) of λ gt 10 EcoRI fragment to make up a whole amount to 10μ $\ell$ . Reaction was effected at 15°C for 16 - 20 hours to synthesize the λ gt 10 DNA vector (concatemer) integrated with the said flounder pituitary gland cDNA.

The recombinant λ gt 10 DNA vector (concatemer) thus obtained was subjected to in vitro packaging by the in vitro packaging extract attached to the kit to produce the flounder pituitary gland cDNA library having an efficiency of 3.3 x 10$^5$ recombinants/ μ g cDNA in E. coli NM514.

Example 7

Selection of Flounder Prolactin cDNA

For selection of the flounder prolactin cDNA from the flounder pituitary gland cDNA library, one employed as probe the synthetic DNA corresponding to the 1st to 13th amino acid sequence in the N-terminal amino acid sequence of the said flounder prolactin (prepared by means of a DNA synthesis apparatus, Synthesizer, Cicron Miligen Ltd.). It has the following base sequence:

$$3'$$
$$\text{CACGGGTAGTT}\genfrac{}{}{0pt}{}{A}{G}\ \text{CT}\genfrac{}{}{0pt}{}{A}{G}\ \text{GAC GAC CT}\genfrac{}{}{0pt}{}{A}{G}\ \text{GCTCGGAGG GT}\genfrac{}{}{0pt}{}{T}{C}\ 5'$$

The said probe was used by marking with [α -$^{32}$P]ddATP and 3′ terminal marking system (NEN/DuPont).

The library (2 x 10$^4$ pfu) obtained by Example 6 was infected with 50 μ $\ell$ of E. coli NM514, which was incubated on LB medium for 16 hours, layered over a LB plate with 9 cm, the DNA of the plaque thus formed was fixed on a nylon filter and plaque hybridization was effected according to the Maniatis et al. method [Molecular Cloning (1982)].

The probe was washed twice with 1XSSC (0.15M NaCl, 0.015 M sodium citrate) 0.1 % SDS liquid at 45°C for every 60 minutes to select the plaque corresponding to that having tightly bound to the said marking probe, whereby there were obtained 20 clones having cDNA of the said flounder prolactin.

Example 8

Determination of Base Sequence of cDNA in Prolactin

From 20 of the recombinant phages obtained by Example 7 was isolated the recombinant λ gt 10 DNA having cDNA of the said flounder prolactin was then isolated by EcoRI treatment.

The resulting cDNA of prolactin was digested with various restriction enzymes to prepare a restriction enzyme map, which was found to be a single common map.

Out of the clones obtained, one having the longest sequence was selected [cfPRL7 (1.3Kb)] and the whole base sequence was determined according to Sanger method [Sanger, F. et al. (1977), Pro. Natl, Acad. Sci. USA, 74, 5463-5467].

The E. coil in which the plasmid containing the DNA fragment cfPRL7 is habored has been deposited as Esherichia coli fPRL7 in the Fermentation Research Institute, Agency of Industrial Science and Technology, Japan, [given accession number FERM BP-3333].

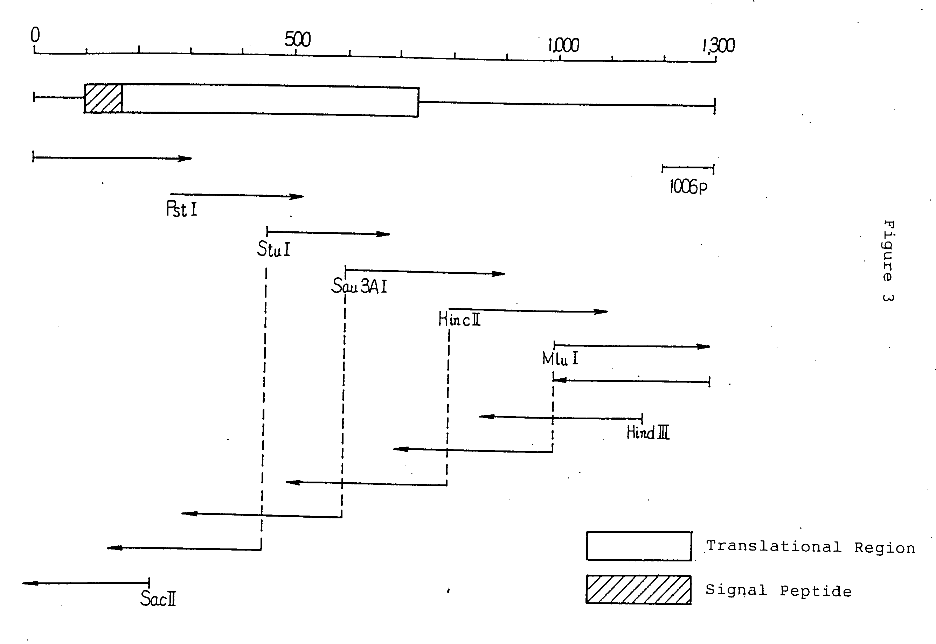

The strategy for determination of base sequence is shown in Fig. 3.

Determination of base sequence was carried out by forming deletion mutants with digestion by restriction enzymes and, if no restriction enzyme site is found, by synthesizing primers.

The base sequence of the flounder prolactin is as shown in Fig. 4.

Of the base sequence as shown in Fig. 4, the sequence of the base number of 1 to 72 codes signal peptide, while the sequence of the base number of 73 to 633 does a mature protein of the said prolactin.

This cDNA base sequence is in complete agreement with the base sequence as expectable from the determined amino acid sequence of the said prolactin, and the said cDNA has been confirmed to code the amino acid sequence of the said prolactin previously determined from the flounder prolactin.

The base sequence of this cDNA proved to have a significant homology with the prolactins discovered in the past.

## Example 9

### Preparation of Recombinant Plasmid Coding for Flounder Prolactin

The plasmid pfPRL7 (5 μ g) containing DNA coding for flounder prolactin was dissolved in 20 μ ℓ of a reaction liquid (hereinafter referred to as "H Buffer") which comprised 100 mM Tris HCl (pH 7.5), 7 mM $MgCl_2$, 50 mM NaCl, 7 mM 2-mercaptoethanol, and 100 μ g/ ml bovine serum albumin,.30 units each of the restriction enzymes EcoRI (Toyobo Co., Ltd., Japan) and BspMI (New England Biolaboratory Co.) was added and reaction was effected at 37°C for 2 hours. The reaction mixture was extracted with phenol, the portion, which coded the amino acid sequence of the flounder prolactin of approximately 1.2 Kbp, was cut out by agarose gel electrophoresis, DNA was recovered by a kit for DNA purification, Geneclean (Funakoshi Yakuhin K.K., Japan) and there was obtained about 0.1 μ g of the fragment of approximately 1.2 Kbp which involved a translation region of mature flounder prolactin coded at cfPRL7 DNA and a 3′ flanking region.

Separately, a prokaryote expression vector (Pharmacia Ltd.) was dissolved in H Buffer, 20 units of the restriction enzyme EcoRI (Toyobo Co., Ltd., Japan) were added and reaction was effected at 37°C for one hour. After precipitation with ethanol, the reaction mixture was dissolved in 50 μ ℓ of a mixture of 67 mM $KPO_4$ (pH 7.4), 6.7 mM $MgCl_2$, 1mM 2-mercaptoethanol, 33 μ M dNTT (dATP, dCTP, dGTP, dTTP), 20 units of Klenow fragment (Toyobo Co., Ltd., Japan) were added and reaction was effected at room temperature for 30 minutes. After addition of one unit of alkaline phosphatase (BAP) (Toyobo Co., Ltd., Japan), was effected at 0°C for 30 minutes. The reaction mixture was extracted with chloroform and DNA was recovered by ethanol precipitation.

Then, two oligonucleotides (31, 31 oligonucleotides) containing initiation codon (ATG) and 5′-terminal GTC (Val) to restriction site were synthesized by using the synthesizer Model 381A (Applied Biosystms Co.).

The oligonucleotide 31 (2 μ g) was dissolved in 50 μ ℓ of a solution of 50 mM Tris HCl (pH 7.6), 10 mM $MgCl_2$, 10 mM 2-mercapto-ethanol and 100 nM ATP, 10 units of T4 polynucleotide kinase (Toyobo Co., Ltd., Japan) was added and reaction was effected at 37°C for 30 minutes. After treatment with phenol, the unreacted ATP was removed by a Sephadex G-50 (Pharmacia Ltd.) column (a volume of 0.9 ml). The phosphonylated oligonucleotide 31 and the previously synthesized oligonucleotide 30 were dissolved in TE buffer [10 mM Tris HCl (pH 7.6), 10 mM EDTA], the solution was heated at 75°C for 5 minutes and then cooled slowly up to room temperature to prepare a DNA linker having the following sequence.

```
EcoRI     Met Val
        ┌──┐┌──┐
AATTC ATG GTC CCC ATC AATG ACC TGC TGGA

     G TAC CAG GGG TAG TTAC TGG ACG ACCT GGCT

                                          BspMI
```

The BspMI-EcoRI fragment of cfPRL7 obtained as above (0.1μ g), the phosphorylated pKK223-3 EcoRI

fragment (0.7 μ g) and the DNA linker (0.5 μ g) were dissolved in 10 μ ℓ of TE buffer and ligation reaction was effected by the use of a ligation kit (Takara Shuzo K.K., Japan). By employing the said reaction liquid, competent E. coli JM109 strain cells (Takara Shuzo K.K., Japan) were transformed and the colonies grown well on LB plates containing ampicillin were selected to obtain the plasmid pKP1 coding the amino acid sequence of mature flounder prolactin.

Example 10

Production of Flounder Prolactin by E. coli containing pKP1

Using the recombinant plasmid pKP1 obatained by Example 9, E. coli JM109 strain was transformed in a conventional manner. The resulting ampicillin-resistant colony was inoculated to 10mℓ of MCG medium (0.6 % Na$_2$HPO$_4$, 0.3 % KH$_2$PO$_4$, 0.5 % NaCl, 0.1 % NH$_4$Cl, 0.5 % glucose, 0.5 % Casamino acid, 1 mM MgSO$_4$, 4 ng/mℓ Vitamin B$_1$, pH 7.2) and incubated at 37°C for 16 hours. The resulting cultured broth was inoculated to 1 ℓ of the MCG medium and incubated at 37°C for 16 hours. Thereafter, the resulting cultured broth was centrifuged at 8,000 rpm for 10 minutes by means of a centrifuge rotor (himac SCR 20B, Hitachi, Ltd., Japan) to recover micro-organisms.

The micro-organisms were suspended in Laemmli Sample Buffer [Laemmli, U. K. (1970), Nature (London), 227, 680-685] and a direct SDS polyacrylamide gel electrophoresis was effected. The Western blotting [Towbin et al., (1979), Proc. Natl. Acad. Sci. USA, 76, 4350] using rabbit antibody was effected for chum salmon prolactin [Kawauchi, et al., (1983), Gen. Comp. Endocrinol, Vol. 49, pp.446-458]. As a result, a band crossing the chum salmon prolactin antibody was observed at the site of a molecular weight of approximately 22,000 daltons. There results can clearly show that E. coli having pKP1 surely produces in a large amount the flounder prolactin.

Example 11

Preparation of Recombinant Flounder Prolactin

A micro-organism capable of producing flounder prodactin, obtained in accordance with Example 10, was incubated to 500 ml of LB medium (1% bactotrypton, 0.5% yeast extract, 0.5% NaCl, pH 7.0) and incubation was allowed to proceed at 37°C for 16 hours. The resulting cultured broth was inoculated to 15 liters of a culture medium comprising 1% glucose, 0.5% yeast extract, 1% bactotrypton, 0.5% NaCl and 45ppm ampicillin, pH 7.0. After incubation at 37°C for 16 hours, the resulting micro-organism was suspended in 20 mM Tris/HCl, 50 mM NaCl pH 7.5, in an amount three times the amount of the micro-organism and then minced by being passed three times through a Manton-Gaulin homogenizer. Then, this suspension was centrifuged at 4°C, by means of a centrifuge rotor (himac SCR20B, Hitachi, Ltd., Japan). The resulting precipitate was washed with distilled water in a amount ten times the amount of the precipitate, again followed by centrifugation under the above conditions to obtain a precipitate. This precipitate was suspended in 20 mM Tris/HCl (pH 8.0), 5 mM EDTA, 0.02% lysozyme, in an amount twenty times the amount of the precipitate, then 10% SDS was added in an amount 0.25 times the amount of the precipitate, then 0.25-fold amount of 10% SDS was added and stirring was performed at 23°C for 1 hour. This solution was then centrifuged at 23°C at 17,000g for 30 minutes and the resulting precipitate was suspended in 20-fold amount of distilled water and washed, again followed by centrifugation under the above conditions afford a precipitate. This precipitate was suspended in 10-fold amount of distilled water and a Tris/HCl, guanidine hydrochloride solution was added to prepare an extraction solution so as to finally satisfy the conditions of 50 mM Tris/HCl, 6M guanidine hydrochloride, pH 8.0. Extraction was carried out at room temperature for 80 hours and the extraction solution was desalted by ultrafiltration using Millpore Pellicon Cassette System (Millipore Co.) and concentrated up to 50 ml. The concentrate was subjected to gel filtration using Sephacryl S-200 (Pharmacia Ltd.) which had been euqilibrated with 50 mM Tris/HCl, 6 M guanidine hydrochloride, pH 8.0. A fraction near approximately 22 KDa in molecular weight resulting from fractional by the gel filtration was diluted with BLM Buffer (0.25% NaHCO$_3$, 0.2% α -lactose, 0.2% mannide, pH 8.5) in an amount twice the amount of the fraction and then dialyzed at 4°C for 24 hours for the BLM Buffer. The dialyzate was centrifuged at 4°C, 26,000g, for 20 minutes, then the supernatant liquid was concentrated again by ultrafiltration, and the concentrate was purified by the reversed-phase HPLC used in Example 2 to afford 20 mg of regenerated recombinant flounder prolactin.

Example 12

Preparation of Recombinant Flounder Prolactin

Following the procedures in Example 10, a micro-organism capable of producing flounder prolactin protein was incubated and harvested, a flounder prolactin protein in inclusion bodies was obtained according to Marston method (DNA cloning; A Practical Approach (Ed. by D. M. Glover), (1987) 3, 59, IRL Press, Oxford). Further, the flounder prolactin protein was extracted according to Schoner et al. method (Bio/Tech (1985), 3, 151), regenerated and purified by HPLC to obtain 20 mg of the recombinant flounder prolactin protein.

Example 13

Determination of Physioloqical Activity

Flounders aged 4 months after hatching (10-15g), a group consisting of 10 fishes, were individually selected and intraperioneally given sheep prolactin (NIAMDD o-PRL-14); the recombinant flounder prolactin obtained by Example 11; and, as a control, physiologial saline every 4 days at 0.01 $\mu$ g and 0.1 $\mu$ g/gram of fish body weight, respectively.

Fish breeding was carried out at 20°C and feed (Nihon Haigoshiryo K.K., Japan) was given in a 1.5 % amount to the body weight of juvenile flounder in a divided from twice mornings and evenings. Body weight gain rates on the 35th day from the first administration are shown in Table 2.

As apparent from Table 2, the prolactin protein according to this invention has been found to be effective in growth promotion of fishes such as flounders and so on.

## Table 2

| Sample | Body weight gain (%) |
|---|---|
| Physiological saline | 1 6 |
| Sheep Prolactin 0.01 $\mu$ g | 2 3 |
| Sheep Prolactin 0.1 $\mu$ g | 2 6 |
| Recombinant Prolactin | 3 0 |
| Recombinant Prolactin 0.1 $\mu$ g | 4 0 |

## Claims

1. A prolactin derived or derivable from the pituitary gland of fishes belonging to the order Pleuronectina, the family Paralichthydae.

2. A prolactin as claimed in claim 1, having the following physicochemical properties;
   (1) Molecular weight: SDS-PAGE about 22,000 daltons,
   (2) Amino acid composition

| Amino acid | Residue number | Amino acid | Residue number |
|------------|----------------|------------|----------------|
| C y s | 4.0 | P r o | 12.5 |
| A s p | 12.4 | T y r | 0.9 |
| G l u | 19.8 | V a l | 7.2 |
| S e r | 24.0 | M e t | 2.7 |
| H i s | 6.3 | I l e | 8.5 |
| G l y | 9.6 | L e u | 27.1 |
| A r g | 11.5 | P h e | 8.3 |
| T h r · | 6.8 | T r p | ND |
| A l a | 16.6 | L y s | 7.5 |

(3) 22 Amino acid residues at N-terminal end having the following sequence:

Val-Pro-Ile-Asn-Asp-Leu-Leu-Asp-Arg-Ala-

Ser-Gln-Arg-Ser-Asp-Gln-Leu-His-Ser-Leu-

Ser-Thr

3. A prolactin as claimed in claim 1 which is obtained by extraction and purification from the pituitary gland of fishes belonging to the order Pleuronectina, the family Paralichthydae.

4. A prolactin as claimed in claim 1 which is a polypeptide obtained by a gene recombination procedure and comprising (a) the following amino acid sequence or (b) a related sequence, provided that said polypeptide of related sequence (b) possesses prolactin activity:

```
Val Pro Ile Asn Asp Leu Leu Asp Arg Ala Ser Gln Arg Ser Asp
 1                               10

Gln Leu His Ser Leu Ser Thr Thr Leu Ser Gln Glu Leu Asp Ser
              20                                         30

His Phe Pro Pro Ile Gly Arg Val Ile Met Pro Arg Pro Ser Met
                                  40

Cys His Thr Ser Ala Leu Gln Thr Pro Asn Asp Lys Thr Gln Ala
              50                                         60

Leu Gln Val Ser Glu Ser Glu Leu Leu Ser Leu Ala Arg Ser Leu
                                  70

Leu Gln Ala Trp Ala Asp Pro Leu Ser Ala Leu Ser Ser Ser Ala
              80                                         90

Phe Ser Leu Pro His Pro Ala Gln Ser Ser Ile Phe Asn Lys Val
                                  100

Arg Glu Met Gln Glu His Ser Lys Asn Leu Gly Asp Gly Leu Asp
              110                                        120

Ile Leu Ser Gly Lys Met Gly Glu Ala Gly Gln Ala Leu Ser Ser
                                  130

Leu Pro Phe Arg Gly Asn Asp Val Gly Gln Asp Arg Ile Ser Lys
              140                                        150

Leu Ile Asn Phe His Phe Leu Leu Ser Cys Phe Arg Arg Asp Ser
                                  160

His Lys Ile Asp Ser Phe Leu Lys Val Leu Arg Cys Arg Ala Ala
              170                                        180

Asn Thr Gln Pro Glu Met Cys
                        187
```

5.    A prolactin as claimed in claim 1 which is a polypeptide comprising the the following amino acid sequence:

```
Met Thr His Arg Arg Thr Lys Leu Phe Met Met Ala Ala Val Val Ser Tyr
-24          -20                                   -10

Val Met Thr Ser Cys Gly Ala Val Pro Ile Asn Asp Leu Leu Asp Arg Ala
                     -1   1                                    10
```

```
Ser Gln Arg Ser Asp Gln Leu His Ser Leu Ser Thr Thr Leu Ser Gln Glu
                                        20

Leu Asp Ser His Phe Pro Pro Ile Gly Arg Val Ile Met Pro Arg Pro Ser
        30                                      40

Met Cys His Thr Ser Ala Leu Gln Thr Pro Asn Asp Lys Thr Gln Ala Leu
                    50                                          60

Gln Val Ser Glu Ser Glu Leu Leu Ser Leu Ala Arg Ser Leu Leu Gln Ala
                                70

Trp Ala Asp Pro Leu Ser Ala Leu Ser Ser Ser Ala Phe Ser Leu Pro His
        80                                  90

Pro Ala Gln Ser Ser Ile Phe Asn Lys Val Arg Glu Met Gln Glu His Ser
            100                                     110

Lys Asn Leu Gly Asp Gly Leu Asp Ile Leu Ser Gly Lys Met Gly Glu Ala
                120

Gly Gln Ala Leu Ser Ser Leu Pro Phe Arg Gly Asn Asp Val Gly Gln Asp
130                               140

Arg Ile Ser Lys Leu Ile Asn Phe His Phe Leu Leu Ser Cys Phe Arg Arg
            150                                     160

Asp Ser His Lys Ile Asp Ser Phe Leu Lys Val Leu Arg Cys Arg Ala Ala
                        170                                     180

Asn Thr Gln Pro Glu Met Cys
                        187
```

6. A gene which (a) codes for an amino acid sequence of a prolactin of fish belonging to the order <u>Pleuro-nectina</u>, the family <u>Paralichthydae</u>, (b) codes for an amino acid sequence related to sequence (a) provided that said related sequence is a sequence of a polypeptide having prolactin activity, or (c) codes for a portion of said amino acid sequences (a) or (b).

7. A gene as claimed in Claim 6 which is DNA coding for the polypeptide comprising (a) the following amino acid sequence or (b) a related sequence provided that said polypeptide of related sequence (b) possesses prolactin activity:

```
Met Thr His Arg Arg Thr Lys Leu Phe Met Met Ala Ala Val Val Ser Tyr
-24             -20                             -10

Val Met Thr Ser Cys Gly Ala Val Pro Ile Asn Asp Leu Leu Asp Arg Ala
                        -1   1                               10.

Ser Gln Arg Ser Asp Gln Leu His Ser Leu Ser Thr Thr Leu Ser Gln Glu
                                20
```

```
Leu Asp Ser His Phe Pro Pro Ile Gly Arg Val Ile Met Pro Arg Pro Ser
        30                                          40

Met Cys His Thr Ser Ala Leu Gln Thr Pro Asn Asp Lys Thr Gln Ala Leu
                    50                                          60

Gln Val Ser Glu Ser Glu Leu Leu Ser Leu Ala Arg Ser Leu Leu Gln Ala
                            70

Trp Ala Asp Pro Leu Ser Ala Leu Ser Ser Ser Ala Phe Ser Leu Pro His
        80                                      90

Pro Ala Gln Ser Ser Ile Phe Asn Lys Val Arg Glu Met Gln Glu His Ser
            100                                         110

Lys Asn Leu Gly Asp Gly Leu Asp Ile Leu Ser Gly Lys Met Gly Glu Ala
                120

Gly Gln Ala Leu Ser Ser Leu Pro Phe Arg Gly Asn Asp Val Gly Gln Asp
130                                     140

Arg Ile Ser Lys Leu Ile Asn Phe His Phe Leu Leu Ser Cys Phe Arg Arg
            150                                     160

Asp Ser His Lys Ile Asp Ser Phe Leu Lys Val Leu Arg Cys Arg Ala Ala
                        170                                     180

Asn Thr Gln Pro Glu Met Cys
                    187
```

8.  A gene as claimed in claim 6 which is DNA coding for the amino acid sequence of the polypeptide containing the amino acid sequence:

```
                                Val Pro Ile Asn Asp Leu Leu Asp Arg Ala
                                 1                                  10

Ser Gln Arg Ser Asp Gln Leu His Ser Leu Ser Thr Thr Leu Ser Gln Glu
                            20

Leu Asp Ser His Phe Pro Pro Ile Gly Arg Val Ile Met Pro Arg Pro Ser
        30                                          40

Met Cys His Thr Ser Ala Leu Gln Thr Pro Asn Asp Lys Thr Gln Ala Leu
                    50                                          60

Gln Val Ser Glu Ser Glu Leu Leu Ser Leu Ala Arg Ser Leu Leu Gln Ala
                            70

Trp Ala Asp Pro Leu Ser Ala Leu Ser Ser Ser Ala Phe Ser Leu Pro His
        80                                      90
```

```
Pro Ala Gln Ser Ser Ile Phe Asn Lys Val Arg Glu Met Gln Glu His Ser
            100                                                 110

Lys Asn Leu Gly Asp Gly Leu Asp Ile Leu Ser Gly Lys Met Gly Glu Ala
                120

Gly Gln Ala Leu Ser Ser Leu Pro Phe Arg Gly Asn Asp Val Gly Gln Asp
130                                     140

Arg Ile Ser Lys Leu Ile Asn Phe His Phe Leu Leu Ser Cys Phe Arg Arg
                150                                 160

Asp Ser His Lys Ile Asp Ser Phe Leu Lys Val Leu Arg Cys Arg Ala Ala
                        170                                     180

Asn Thr Gln Pro Glu Met Cys
                    187
```

9. A gene as claimed in claim 6 which is DNA having the following base sequence or a portion thereof:

```
            10              20              30              40              50
ATG ACT CAC AGA AGA ACC AAA CTC TTC ATG ATG GCT GCA GTT GTG TCG TAC

            60              70              80              90              100
GTG ATG ACA TCA TGC GGC GCC GTC CCC ATC AAT GAC CTG CTG GAC CGA GCG

            110             120             130             140             150
TCT CAG CGT TCG GAC CAA CTG CAC TCG CTC AGC ACG ACA CTC AGC CAA GAG

            160             170             180             190             200
CTG GAC TCT CAT TTC CCT CCT ATT GGT CGG GTG ATC ATG CCG CGG CCC TCC

            210             220             230             240             250
ATG TGC CAC ACC TCC GCT CTG CAG ACG CCC AAT GAC AAG ACT CAG GCT CTT

            260             270             280             290             300
CAA GTA TCG GAG TCC GAG CTG TTG TCC CTG GCT CGC TCT CTG CTC CAG GCC

            310             320             330             340             350
TGG GCC GAC CCT CTG TCC GCC CTG TCC TCC TCG GCC TTC TCT CTG CCT CAC

            360             370             380             390             400
CCG GCA CAA AGC AGC ATA TTC AAC AAG GTG CGC GAG ATG CAG GAG CAC TCC

410             420             430             440             450
AAG AAC CTG GGG GAC GGC CTG GAC ATC CTC TCT GGG AAA ATG GGT GAG GCG

460             470             480             490             500             510
GGG CAG GCT CTC TCC TCC CTG CCC TTC AGA GGC AAC GAC GTC GGC CAG GAC

            520             530             540             550             560
AGG ATT TCC AAA CTG ATC AAC TTC CAC TTC CTG TTG TCC TGC TTC CGA CGG
```

```
              570              580              590              600              610
   GAC TCC CAC AAG ATT GAC AGC TTC CTG AAA GTC CTG CGC TGC CGG GCT GCA
              620         630    636
   AAC ACG CAA CCT GAG ATG TGC TGA
```

**10.** A gene as claimed in claim 6 which is a synthetic DNA corresponding to the amino acid sequence from N-terminal end to 13th of the amino acid sequence of prolactin derived from the pituitary gland of fishes belonging to the order Pleuronectina, the family Paralichthydae.

**11.** A recombinant vector integrated with a gene coding for the amino acid sequence of prolactin of fishes belonging to the order Pleuronectina, the family Paralichthydae or a related sequence as defined in Claim 3.

**12.** A recombinant vector as claimed in Claim 11, integrated with a gene as claimed in any of Claims 7 to 10.

**13.** A transformant containing a recombinant vector integrated with a gene which (a) codes for the amino acid sequence of prolactin of fishes belonging to the order Pleuronectina, the family Paralichthydae, (b) codes for a related sequence as defined in Claim 3 or (c) codes for a portion thereof.

**14.** A transformant as claimed in Claim 13 which contains a recombinant vector as claimed in Claim 12.

**15.** A transformant as claimed in Claim 13 which is a microorganism.

**16.** A transformant as claimed in Claim 15 which belongs to E. coli.

**17.** A process for producing a prolactin of fishes belonging to the order Pleuronectina, the family Paralichthydae which comprises incubating and multiplying a transformant transformed by a recombinant vector, said vector being integrated with a gene coding for the amino acid sequence of said prolactin, and recovering the said prolactin protein as produced.

**18.** A process of growth promotion for animals which comprises administering, preferably by feeding, a prolactin of fishes belonging to the order Pleuronectina, the family Paralichthydae to said animal to promote growth thereof.

**19.** A process as claimed in Claim 18 wherein said prolactin is the protein as claimed in any of Claims 2 to 5.

**20.** A feed for animals, comprising a prolactin of fishes belonging to the order Pleuronectina, the family Paralichthydae.

**21.** A prolactin as claimed in any of Claims 1 to 5 having an amino sequence which differs from the amino acid sequence set forth in Claim 5 by not more than 20, preferably not more than 10 and most preferably not more than 5 amino acid insertions, deletions and/or substitutions.

**22.** A gene as claimed in Claim 7 wherein said related sequences are as defined in Claim 21

**23.** A synthetic DNA probe as claimed in Claim 10 having the base sequence

$$3' \quad \text{CACGGGTAGTT}\binom{A}{G}\text{CT}\binom{A}{G}\text{GAC GAC CT}\binom{A}{G}\text{GCTCGGAGG GT}\binom{T}{C} \quad 5'$$

**24.** A gene as claimed in claim 6 obtained by probing a gene bank using a synthetic DNA as claimed in Claim 10 or Claim 23.

Figure 1

Figure 2

Figure 3

Figure 4

```
          10              20              30              40              50
ATG ACT CAC AGA AGA ACC AAA CTC TTC ATG ATG GCT GCA GTT GTG TCG TAC
Met Thr His Arg Arg Thr Lys Leu Phe Met Met Ala Ala Val Val Ser Tyr
-24             -20                                             -10
          60              70              80              90             100
GTG ATG ACA TCA TGC GGC GCC GTC CCC ATC AAT GAC CTG CTG GAC CGA GCG
Val Met Thr Ser Cys Gly Ala Val Pro Ile Asn Asp Leu Leu Asp Arg Ala
                         -1  1                                          10
         110             120             130             140             150
TCT CAG CGT TCG GAC CAA CTG CAC TCG CTC AGC ACG ACA CTC AGC CAA GAG
Ser Gln Arg Ser Asp Gln Leu His Ser Leu Ser Thr Thr Leu Ser Gln Glu
                                         20
         160             170             180             190             200
CTG GAC TCT CAT TTC CCT CCT ATT GGT CGG GTG ATC ATG CCG CGG CCC TCC
Leu Asp Ser His Phe Pro Pro Ile Gly Arg Val Ile Met Pro Arg Pro Ser
         30                                              40
         210             220             230             240             250
ATG TGC CAC ACC TCC GCT CTG CAG ACG CCC AAT GAC AAG ACT CAG GCT CTT
Met Cys His Thr Ser Ala Leu Gln Thr Pro Asn Asp Lys Thr Gln Ala Leu
                 50                                              60
         260             270             280             290             300
CAA GTA TCG GAG TCC GAG CTG TTG TCC CTG GCT CGC TCT CTG CTC CAG GCC
Gln Val Ser Glu Ser Glu Leu Leu Ser Leu Ala Arg Ser Leu Leu Gln Ala
                                 70
         310             320             330             340             350
TGG GCC GAC CCT CTG TCC GCC CTG TCC TCC TCG GCC TTC TCT CTG CCT CAC
Trp Ala Asp Pro Leu Ser Ala Leu Ser Ser Ser Ala Phe Ser Leu Pro His
         80                                      90
360             370             380             390             400
CCG GCA CAA AGC AGC ATA TTC AAC AAG GTG CGC GAG ATG CAG GAG CAC TCC
Pro Ala Gln Ser Ser Ile Phe Asn Lys Val Arg Glu Met Gln Glu His Ser
                 100                                             110
410             420             430             440             450
AAG AAC CTG GGG GAC GGC CTG GAC ATC CTC TCT GGG AAA ATG GGT GAG GCG
Lys Asn Leu Gly Asp Gly Leu Asp Ile Leu Ser Gly Lys Met Gly Glu Ala
                 120
460             470             480             490             500             510
GGG CAG GCT CTC TCC TCC CTG CCC TTC AGA GGC AAC GAC GTC GGC CAG GAC
Gly Gln Ala Leu Ser Ser Leu Pro Phe Arg Gly Asn Asp Val Gly Gln Asp
130                                              140
         520             530             540             550             560
AGG ATT TCC AAA CTG ATC AAC TTC CAC TTC CTG TTG TCC TGC TTC CGA CGG
Arg Ile Ser Lys Leu Ile Asn Phe His Phe Leu Leu Ser Cys Phe Arg Arg
                 150                                             160
         570             580             590             600             610
GAC TCC CAC AAG ATT GAC AGC TTC CTG AAA GTC CTG CGC TGC CGG GCT GCA
Asp Ser His Lys Ile Asp Ser Phe Leu Lys Val Leu Arg Cys Arg Ala Ala
                                 170                                     180
         620             630     636
AAC ACG CAA CCT GAG ATG TGC TGA
Asn Thr Gln Pro Glu Met Cys ***
                         187
```

Figure 5